# EUROPEAN PATENT SPECIFICATION

(11) **EP 0 689 596 B1**
(45) Date of publication and mention of the grant of the patent: **07.11.2001**
(21) Application number: 94909182.1
(22) Date of filing: 11.03.1994
(51) Int. Cl.: C12N 15/45, A61K 35/76, A61K 48/00, A61K 39/155, C12N 9/24, C12N 15/56

(54) **STIMULATION OF IMMUNE RESPONSE BY VIRAL PROTEIN**
STIMULIERUNG DER IMMUNANTWORT DURCH VIRALES PROTEIN
STIMULATION D'UNE REPONSE IMMUNITAIRE PAR UNE PROTEINE VIRALE

(30) Priority: 16.03.1993 EP 93301962
(43) Date of publication of application: 03.01.1996
(73) Proprietor: Von Hoegen, Paul, 1380 Lasne/Ohain (BE); Schirrmacher, Volker, Prof. Dr., 69117 Heidelberg (DE)
(72) Inventor: ERTEL, Christian, D-68163 Mannheim (DE); VON HOEGEN, Paul, B-1380 Lasne/Ohain (DE); SCHIRRMACHER, Volker, D-69117 Heidelberg (DE)
(74) Representative: Schüssler, Andrea, Dr.
(86) International application number: GB9400476
(87) International publication number: WO9421798

(56) References cited:
- EP-A- 0 227 414
- EP-A- 0 420 759
- US-A- 5 124 148
- EUR. J. IMMUNOL. vol. 18 , 1988 pages 1159 - 1166 P. VAN HOEGEN ET AL. 'Modification of tumor cells by a low dose of Newcastel Disease Virus' cited in the application
- CANCER IMMUNOL. IMMUNOTHER. vol. 35 , 1992 pages 325 - 330 P. SCHLAG ET AL. 'Active specific immunotherapy with NDV-modified tumor cells following resection of liver metastases in colorectal cancer' cited in the application
- VIROLOGY vol. 171 , 1989 pages 10 - 17 T. MORRISON ET AL. 'Avian cells expressing the NDV haemagglutinin-neuraminidase protein are resistant to NDV infection'
- EUROP. J. IMMUNOL. vol. 23 , 1993 pages 2592 - 2596 C. ERTEL ET AL. 'Viral hemagglutinin augmentds peptide-specific cytotoxic T cell responses'

## Description

### Background of the invention

### 1. Field of the invention

This invention is in the field of immunology and relates to the use of a certain viral protein, as expressed from viral cDNA, to stimulate one particular form of immune response, namely to increase an antigen-specific cytotoxic lymphocyte (CTL) response.

### 2. Description of the related art

A cell-mediated immune response to a pathogenic antigen has several stages. In the first stage, the pathogen is engulfed by a macrophage. The macrophage processes the antigen in small fragments and presents it to a T-cell. Such macrophages are known as antigen-presenting cells (APCs). The T-cell recognises the antigen as foreign through the intervention, at this stage, of a major histocompatibility complex antigen. The T-cell, which is a lymphocyte, is assisted by interleukin-1, produced by the macrophage, to stimulate the formation of functionally specialised T-cells. These include helper T-cells which have a phenotype described as CD4⁺. The helper cells, with the aid of interleukin-2, stimulate the production of killer T-cells, also known as cytotoxic T-lymphocytes (CTLs). They have a phenotype described as CD8⁺. The CTLs are antigen-specific. When they recognise the foreign antigen, they kill the cell containing it by lysis.

The natural feedback mechanisms of the human body will increase the number of functional CTLs available to combat the particular foreign antigen which has invaded the body. However, it is sometimes desirable in therapy to increase the cell-mediated response to a particular antigen.

Antigen specific CTL responses can be increased by cytokines such as gamma interferon [M.M. Simon et al., J. Immunol. 136, 2755-2762 (1986)], or alpha/beta interferon (L.K. Chen et al., Eur. J. Immunol. 16, 767-770 (1986); P. von Hoegen et al., Cellular Immunology 126, 80-90 (1990)] or by modification of the antigen-presenting cells (APCs), by immunising mice with antigen-containing cells infected with the poultry virus, Newcastle Disease Virus (NDV) [P. von Hoegen and V. Schirrmacher, Annals N.Y. Acad. Sci. 532, 468-471 (1988); P. von Hoegen et al., J. Immunol. 18, 1159-1166 (1988)]. The treatment of patients suffering from colorectal cancer with cancer cells taken from the patient and treated with NDV is disclosed by W. Liebrich et al., Eur. J. Cancer 27, 703-710 (1991) and P. Schlag et al., Cancer Immunology and Immunotherapy, 35, 325-330 (1992).

It would be desirable to find other means of increasing antigen-specific CTL responses. Cytokines would have to be administered in a large amount which raises problems of toxicity. Infection with whole NDV raises difficulties of safety and reproducibility, considering that the virus is grown in chicken eggs and also the possible actions of different component proteins. Further prior art, the relevance of which becomes clear only with the knowledge of the invention, is mentioned after the "Summary of the invention" section.

### 3. Summary of the invention

According to the present invention, there is provided the new medical use of DNA encoding the haemagglutinin-neuraminidase (HN) protein of a paramyxovirus, especially NDV, of its native paramyxovirus, and free of any associated specific antibody to the HN protein, for the purpose of increasing the stimulation of antigen-specific T cell responses in humans. The same use of DNA coding for a peptide derivable from the HN protein and having the stimulation-increasing effect, is also included in the invention. The invention may be expressed in various ways according to the requirements of domestic patent law, including in Europe the use for the manufacture of a medicament for the above purpose and in the United States a method of providing the said increase in stimulation which comprises administering to the human patient an amount of the DNA effective for the said purpose. Normally, the HN DNA would be administered in the form of cells infected with or already expressing the foreign antigen and infected by the HN DNA. The term "infection" is used in this context in a broad sense to include procedures such as electroporation or calcium phosphate precipitation, often called "transfection", as well as infection using a viral vector for the DNA, as in a retrovirus or poxvirus, for example. The terminology "free of its native paramyxovirus" simply clarifies that it is not intended to cover the use of whole particles of NDV or other paramyxovirus.

In a first possibility, cells are taken from a patient suffering from a disease, for example tumour cells. These cells are inactivated, e.g. by irradiation, infected with the HN DNA and then administered back to the patient. In a second possibility, the invention is used more generally for vaccination. Macrophages or other APCs, e.g. any HLA class I or II-expressing cells, are isolated from a patient or are grown in culture and matched with the patient's MHC, incubated with a T-cell epitope-bearing peptide or other suitable antigen and infected with the HN DNA. The invention therefore further provides a vaccine suitable for use in combatting a disease in a human patient, to which a T-cell mediated immune response can be mounted, the vaccine comprising antigen-presenting cells (APCs) tolerable to the patient, presenting an antigen of high specificity for the disease and containing an HN protein-providing component comprising DNA coding for the HN protein of a paramyxovirus, or a T-Cell stimulation-enhancing peptide thereof, whereby the in vivo stimulation of cyotoxic T-cells of high specificity for cytolysis of diseased cells is increased by the HN protein-providing component. In one aspect, the APCs are provided by inactivated diseased cells previously removed from the patient to be treated or from a donor patient of matched MHC type.

### Further description of prior art

Among many possible approaches, the inventors chose to proceed further with that based on NDV. NDV is an avian paramyxovirus with a single strand RNA of negative polarity approximately 15 kb in length coding for six genes (3'-NP-P-M-F-HN-L-5'). The HN of NDV is a 74 kD membrane glycoprotein which protrudes from the viral envelope and has both hemagglutinating (HA) and neuraminidase activity (NA) [N.S. Millar et al., J. Gen. Virol. 67, 1917-1927 (1986)]). HN protein plays a key role in mediating the attachment of virus to host cell receptors. However it does not serve as a major target for the cellular immune response of the host, which most likely is directed against the NP, as seen with the influenza virus.

There have been suggestions that neuraminidase is implicated in immune responses. R. L. Simmons and A. Rios, Cancer, 34, 1541-1547 (1974) reported that firmly established tumours in syngeneic mice will regress if challenged with living tumour cells which have been exposed in vitro to Vibrio cholerae neuraminidase (VCN). It was suggested that neuraminidase acts on the cell surfaces to remove terminal sialic acid residues. However, the authors could not explain satisfactorily a similar effect on the tumours when conconavalin A was used instead of VCN. Fifteen years later, it was found that chicken cells transfected with retrovirus containing the NDV HN gene were resistant to infection by NDV and by influenza virus. It was again speculated that this was due to the destruction of cell receptors by the neuraminidase: T.G. Morrison and L.W. McGinnes, Virology 171, 10-17 (1989). However, these authors did not suggest any more general action of the HN gene to augment immune response.

U.S. Patent 5,124,148 (Csatary and Massey, assignors to United Cancer Research Institute) claims a method of significantly increasing the numbers of T-4 helper-inducer T cells, white blood cells and platelets in humans who have AIDS by administering to them 1000 units of attenuated avian paramyxovirus, such as NDV ("strain H") by intramuscular injection once a week for at least four weeks. Nothing is said about increasing the activity of the T-cells. The specification also describes other immunological effects of avian paramyxoviruses, including the survival of mice infected with a pathogenic lymphoma virus when co-inoculated with NDV. The specification asserts that other viral forms other than whole attenuated virus preparations are usable, referring to "viral components, such as purified RNA", but no details are given. Thus, it gives no hint that the HN gene of NDV would augment the immune response, nor is it obvious that it would do so merely from the above-mentioned prior speculation that neuraminidase might destroy certain cell receptors.

R. E. Randall and D. F. Young, J. Gen. Virol. 69, 2505-2516 (1988) have described a procedure of preparing monoclonal antibodies specific to a Simian Virus SV5 protein, including the HN protein, complexing these antibodies to a solid matrix (a suspension of Staphylococcus aureus Cowan A strain) and using the complex to immunopurify the SV5 protein. The resultant solid matrix-antibody-SV5 HN antigen (SMAA) complexes were used to immunise mice. They induced both humoral and cell-mediated responses. The cell-mediated response was not compared with that which might be obtained from uncomplexed SV5 HN. The paper interprets the results as attributable to the SMAA method of presentation and in no way suggests that the HN protein might itself be a T cell stimulator. Presenting the SV5 HN as a complex with a specific antibody thereto is not very practical, because of additional problems in relation to the safety of use of such antibodies and is therefore excluded from the scope of this invention.

### Brief Description of the drawings

Fig. 1 illustrates the procedure for introducing an additional BamHI restriction site, to facilitate use of the plasmid pSVL-HN.1 (which is readily derived from known plasmids), and for its insertion into the BamHI site of an expression plasmid p36/7;
Fig. 2 illustrates in more detail the plasmid p 36/7-HN.2, being the plasmid into which NDV HN cDNA has been inserted;
Figs. 3A-3C show results of measurement of expression of HN and of neuraminidase activity in mouse Ltk⁻ cells transfected with NDV HN cDNA.
Fig. 4 shows the peptide-specific cytotoxic effect compared with the non-specific cytotoxic effect at various different ratios of effector cells (mouse Ltk⁻ cells preincubated with a peptide and with NDV HN) to target mouse Ltk⁻ cells preincubated with peptide cells; and
Fig. 5 shows the stimulation index in lytic units per 10⁶ cells when mice having various specific cells are inoculated with these cells, with and without pre-transfection with NDV.

### Description of the preferred embodiments

In one aspect, the invention requires the use of viral cDNA encoding a HN protein. This can be simply a copy of the genomic RNA or it can have minor variations, either within the degenerancy of the genetic code so that it codes for the same sequence of amino acids or variations which result in minor changes to amino acids, whereby the protein functions are not altered adversely. It is contemplated that the full length DNA will normally be used, i.e. that coding for the entire HN gene, but, clearly, it is a simple matter to find shorter, but still usable lengths of the gene by experiment. It would also be possible to make a fusion protein in which the HN protein or some part thereof is fused to a protein having a cell surface binding capacity, e.g. for binding to selected cells especially those which do not express sialic acid in sufficient amounts. For example, it could have fused to it an epidermal growth factor (EGF) protein which could bind to the EGF receptor of mammary cells.

Hereinafter, the invention will be described largely by reference to the HN protein of NDV, but it will be appreciated that there are other paramyxoviruses which bear a close degree of homology in. their HN protein with that of NDV. The invention includes the use of such proteins and the DNA encoding them. Examples are Sendai virus, the HN cDNA of which has been cloned by N. Miura et al.. FEBS 188 (1), 112-116 (1985) and human paramyxovirus or parainfluenza virus.

In particular, the invention includes the use of HN proteins which have a degree of homology (identity) with the HN protein of NDV Beaudette C strain of at least 70% at the amino acid level and, more preferably, at the level of cDNA encoding the protein. This definition includes, of course, virtually all strains of NDV, such as Hitchin, Ulster 2C, Victoria, Italien etc.

Immunisation can be carried out by giving the patient an inoculation, e.g. by intravenous injection, with the patient's own cells, especially cells comprising macrophages, to avoid graft/host rejection or other cells tolerable for the patient, which contain the antigen of interest and the HN DNA. Preferably the HN DNA is introduced along with a retroviral vector containing the HN DNA, see J. Price et al., Proc. Natl. Acad. Sci. USA 84 156-160 (1987). Alternatively, the cells are transfected by DNA from an appropriate plasmid. For this purpose, the DNA is inserted into a eukaryotic cell expression vector under the control of a suitable promoter, for example a metallothionein promoter. Preferably the vector also contains at least one drug resistance marker for use in eukaryotic cells. The tolerable cells are then transfected with the HN DNA by the calcium phosphate precipitation method. Alternatively, the HN DNA could be incorporated in an adenovirus vector which could be injected directly into a tumour or other infected area of the body, see B. Quantin et al., Proc. Natl. Acad. Sci. USA 89, 2581-2584 (1992).

The vaccines of the invention can be formulated in any usual way and may include an adjuvant. The invention also includes the preparation of vaccines by appropriate infection of APCs by DNA or incubation with the relevant antigen, as described above and/or in the Examples.

A dose of DNA expressing the HN protein in the range of 10-100, preferably 20-60 HU/10⁷ APCs is suggested. This is consistent with the dose of 32 HU/10⁷ cells used in a phase II trial of whole NDV, see P. Schlag et al. cited above.

The results presented here demonstrate a new finding, namely that expression of the HN-gene product in APCs can increase their antigen-presenting capacity. This becomes especially applicable in weak immune responses such as secondary responses against peptides and tumour antigens.

The invention is primarily of interest in relation to cancer antigens, some of which have poor action as antigens in stimulating immune responses. One example is the tumour-associated antigen, analogous to that produced in mice by the highly metastatic ESb lymphoma. Another is the human melanoma antigen described by P. Van der Bruggen et al., Science 254, 1643-1647 (1991). Other examples of antigens arising from point mutations of non-carcinogenic proteins will be evident from that paper. Among non-cancer antigens, listeria antigens and viral antigens, especially gp 120 or 160 of HIV-1, are of particular interest.

Another use of the invention is in screening peptides for their capacity to act as antigens, by pulsing the APCs with the HN DNA and then testing their ability to stimulate immune cells which recognise one particular antigen of interest.

The following Examples illustrate the invention.

### EXAMPLE 1

### (a) Construction of the eukaryotic plasmid vector p 36/7-HN.2

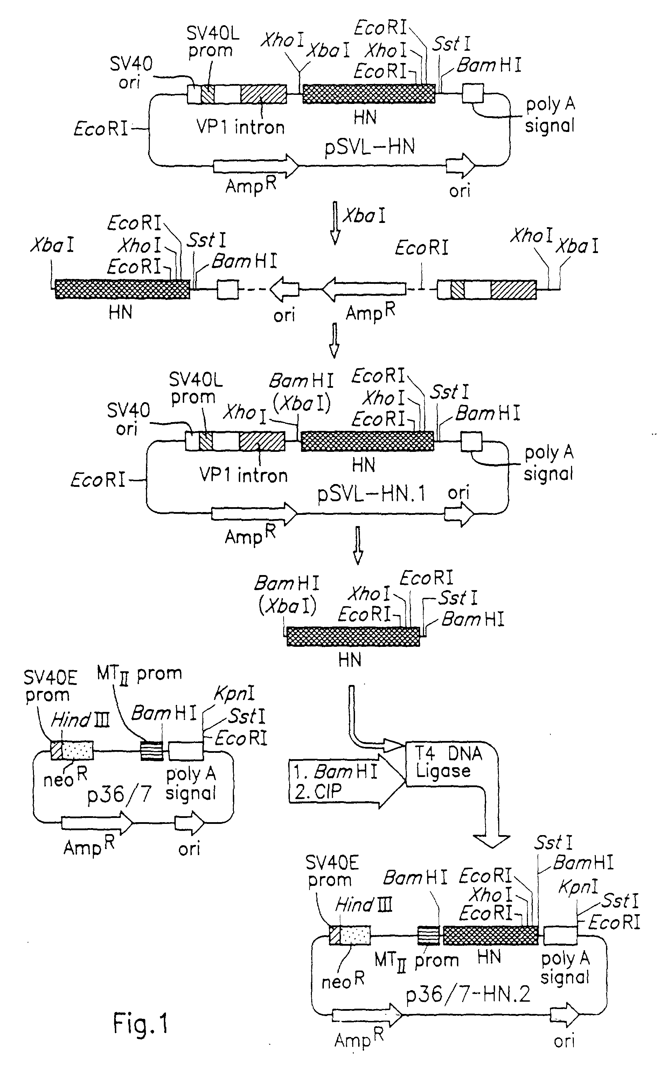

The construction of a clone of the full length NDV (Beaudette C. strain) HN gene in plasmid pUC19 in E. coli has been described previously [P. Chambers et al., J. Gen. Virol. 69, 2115-2122 (1988); European Patent EP-B 227 414 (British Technology Group Ltd.)]. This clone in E. coli of a plasmid (pUC19) containing the full length HN NDV DNA has been deposited at the National Collections of Industrial and Marine Bacteria (NCIMB), 23 St. Machar Drive, Aberdeen AB2 1RY, Scotland, under the provisions of the Budapest Treaty on the International Recognition of the Deposit of Micro-organisms for the Purposes of Patent Procedure on 1st July 1986 under the number NCIMB 12278. This deposit can be accessed already via the European Patent Office in connection with EP-B 227 414. The clone, which was derived from viral genomic RNA, contains the 3' region of the NDV F gene upstream of the HN gene. At the 5' end of the clone is an SphI site [position 1610-1615 in the sequence reported in EP-B 227 414 or 1-6 in N.S. Millar et al., J. Gen. Virol 67, 1917-1927 (1986)], which is 304 nucleotides upstream of the HN start codon. The clone extends to an SstI site [position 3733-3738 in EP-B 227 414 or 2124-2129 in Millar et al. (1986)] which is 87 nucleotides downstream from the end of the HN coding sequence. The 3' noncoding sequence and F coding sequence of this clone contains seven additional potential initiation codons. To enable the efficient expression of the HN gene, this upstream sequence was removed by exonuclease digestion. The plasmid was linearised at its unique SphI 3' cloning site and 25 µl of linearised plasmid incubated at 37°C with 1 unit Bal31 in 200 µl of a buffer containing 0.6 M NaCl, 12 mM CaCl₂, 12mM MgCl₂, 0.2 mM EDTA and 20 mM Tris-HCl, pH 8.0. Aliquots (20 µl) were removed at times from 30 seconds to 15 minutes and the reaction stopped by the addition of each aliquot to 2 µl of 200 mM EDTA and then inactivated by heating at 65°C for 5 minutes. The ends of the digested DNA were repaired by filling in with the Klenow fragments of DNA polymerase 1 (1 unit) in the presence of all four dNTPs (each 5 µM). After heat inactivation, the truncated plasmid was cut with SstI. The deleted fragments from each of the time points were separately ligated into M13mp18 at the SmaI and SstI sites in the M13 multiple cloning site. After transformation into E. coli, individual plaques from each time point were isolated and their insert sequenced. A series of deletions from the 3' SphI site to points throughout the HN gene were obtained. An M13 recombinant (M13-HN10) was identified which was deleted to a point 101 nucleotides upstream of the HN translational start site and from which all upstream potential initiation codons had been removed. The insert from M13-HN10 was excised with XbaI (in the M13 multiple cloning site) and SstI (at the 5' end of the HN cDNA) and subcloned into the XbaI and SacI (SstI) sites in the multiple cloning site of the 4.9 kb plasmid expression vector pSVL (Pharmacia) to give the plasmid pSVL-HN, which is shown schematically in Figure 1 of the drawings and has a size of about 6.7 kb.

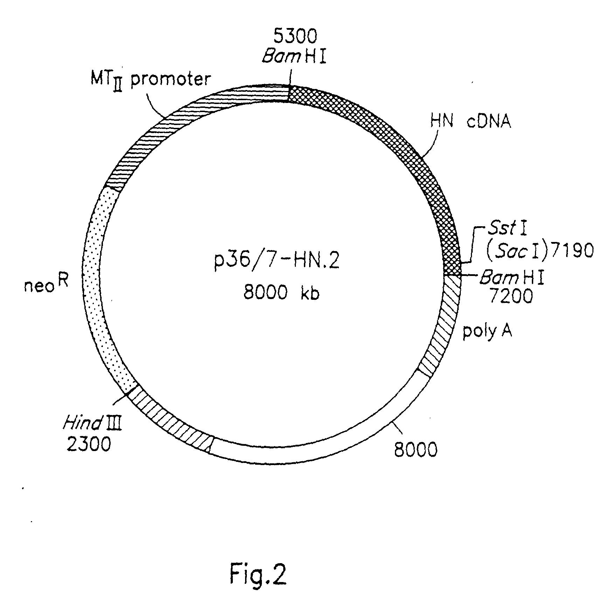

Referring to Fig. 1, for better expression, the pSVL-HN plasmid was digested with XbaI which cuts the plasmid just upstream of the HN sequence. The XbaI site was filled in and converted into a BamHI site. The HN gene was then excised from the plasmid by BamHI which cuts upstream and downstream of the HN gene and ligated to a BamHI-cut plasmid p36/7 and re-circularised to produce the plasmid p36/7-HN.2, which is made up from a SV40E promoter, neomycin resistance gene (from a pSV-neo plasmid), a metallothionein (MT_{II}) promoter, the HN gene and a poly-A signal. Figure 2 shows this plasmid p36/7-HN.2 on a larger scale and in more detail.

### b) Demonstration that mouse fibroblast cells transfected with NDV HN cDNA express HA and NA

The mouse fibroblast cell line Ltk⁻ described by S. Kit et al., Exp. Cell. Res. 37, 291-312 (1963) provides cells which function as antigen-presenting cells (APCs) having the correct major histocompatibility complex (MHC) for recognition by the T-cells of the mice used in subsequent experiments. Ltk⁻ cells were plated out at a density of 10⁶ cells per dish 24 hours before use for transfection. Calcium phosphate precipitates of DNA (20 µg) from the plasmid p36/7-HN.2 were prepared as described by M. Wigler et al., Cell 14, 725-731 (1978). The Ltk⁻ cells were exposed to the precipitated DNA for 6 hours and then shocked for 3 minutes with Hanks' buffered saline (HBS) medium containing 15% glycerol. After 24 hours, the transfected cells were selected with G418 at 600 µg/ml. After 2-3 weeks, G418-resistant colonies were cloned by limiting dilution.

The NDV HN cDNA-transfected cells were detached from culture plates by treatment with EDTA, washed twice and incubated for 20 minutes at 4°C with monoclonal antibodies (8C11) specific for NDV HN protein or, as controls, with monoclonal antibodies (12C4) specific for the NDV F protein or with PBS. Both monoclonal antibodies were kindly donated by D. Meulemans (Brussels), see L. Long et al., J. Virol. 57, 1198-1202 (1986). The cells were then washed twice with PBS/2% fetal calf serum (FCS). Fluorescein isothiocyanate (FITC)-conjugated goat antibodies to mouse Ig, diluted 1/100 in 50 µl PBS/2% FCS, were added over 30 minutes at 4°C. The cells were washed twice in phosphate buffered saline (PBS) and analysed with a FACScan fluorescence analyser (Becton Dickinson). In this analysis, the relative fluorescence intensity per cell was calculated from 10,000 cells using the LYSIS II software.

To assay for neuraminidase, a new fluorimetric method was developed. The neuraminidase-expressing Ltk⁻ cells were developed. The cells expressing neuraminidase were coincubated at 10⁷ cells/ml with sheep red blood cells (SRBC) (0.1%) in PBS for 1 hour at 37°C. The neuraminidase unmasked receptors for peanut agglutinin (PNA) present on the surface of the SRBC. For detection of the unmasked receptors, the cells were washed twice and incubated with FITC-labelled PNA (3.125 mg/ml) for 20 minutes at room temperature. After two washings, the SRBC were analysed by flow cytometry in the above-described analyser. Ltk⁻ cells were excluded from analysis by a combination of forward and side scatter gating.

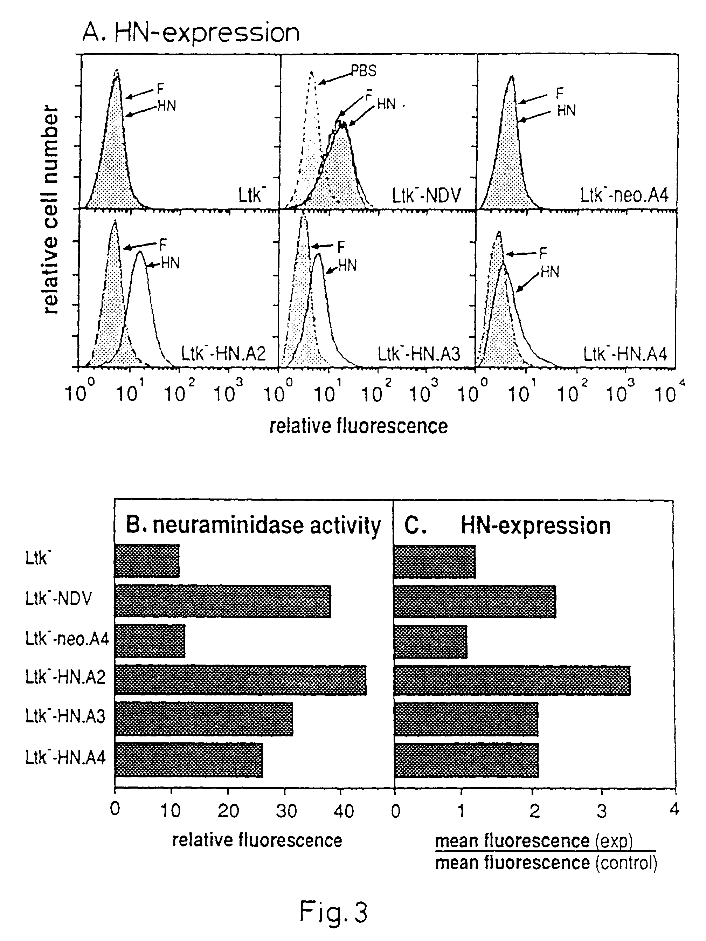

Results are shown in Fig. 3. To show the relation between HN-expression (Fig. 3A) and neuraminidase activity (Fig. 3B), the results of the HN-expression analysis of Fig. 3A are re-presented in Figure 3C in terms of fluorescence expressed as a ratio to the mean fluorescence of the experimental groups (anti-HN) to that of the appropriate controls. The controls were (a) the PBS treated one for the Ltk⁻ NDV (whole virus) and (b) the Ltk⁻ cells incubated with anti-F protein antibody for the Ltk⁻ NDV HN cDNA transfected group. The expression of both parameters was compared with mock-infected Ltk⁻ cells (Ltk-neo.A4) and Ltk⁻ cells infected with 100 haemagglutinin units (HU)/10⁷ cells of whole NDV of strain Ulster 2C grown in the allantoic cavity of embryonated chicken eggs (Ltk⁻ NDV). Three clones, Ltk⁻-HN. A2, A3 and A4, selected from the cytometry analysis, expressed both HN and NA, as can be seen from the relative activities shown in Figure 3B and 3C. Clone Ltk⁻-HN.A2 shows highest expression of HN and NA, which is even above the activity seen with whole NDV-infected Ltk⁻ cells. The two other clones (Ltk⁻-HN.A3 and Ltk⁻-HN.A4) show a weaker but still significant expression.

### c) Preparation of antigen (peptide) specific antigen-presenting cells

The mouse Ltk- cells were removed from culture by treatment with EDTA and suspended at 10⁷ cells/ml. in medium RPMI (Gibco BRL) containing 10% FCS (Gibco BRL) containing 10 µg/ml of the influenza nucleoprotein peptide 50-63 (NP 50-63). After incubation for 1 hour at 37°C, the cells were washed twice with fresh medium.

### d) Demonstration that expression of NDV HN cDNA enhances an antigen (peptide)-specific stimulation of CTL

C3H mice were Immunised with 100 µg of peptide NP 50-63 in 50µl Freund's Incomplete Adjuvant (FIA) in the base of the tail. 10 days later, the spleen cells were restimulated in vitro with Ltk⁻ cells that had been preincubated for 1 hour at 37°C in medium containing 10 µg/ml of peptide NP 50-63 and transfected with NDV HN cDNA or infected with whole NDV as described previously. The re-stimulated spleen cells contain T-cells which include cytotoxic T-cells made specific to the NP 50-63 peptide. These cells are termed "effector cells". The peptide-specific cytotoxicity of the effector cells was measured in a standard 4-h ⁵¹Cr-release assay against Ltk⁻ "target cells" which had been ⁵¹Cr labelled and then preincubated, with or without peptide NP 50-63, as described above in section (c) for the APCs. Various different ratios of cell numbers of effector to target cells were used, viz. 50:1, 25:1, 12:1 and 6:1.

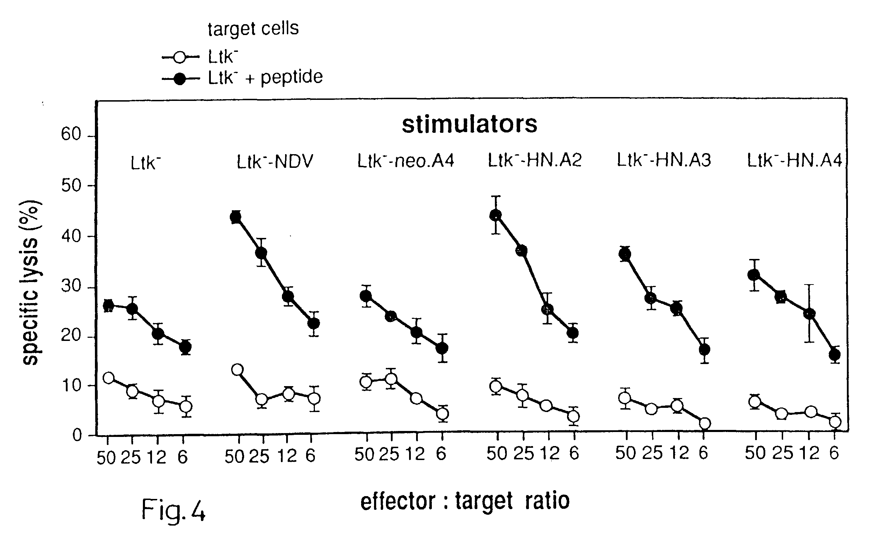

The results are shown in Fig. 4 of the drawings in which cell lysis is shown for the various different experiments. All three HN-expressing Ltk⁻-HN clones induced a stronger activation of peptide-specific CTL activity than the Ltk⁻ and Ltk⁻-neo. controls. For Ltk⁻-HN.A2 the enhancement was indistinguishible from that of whole NDV-infected Ltk⁻ cells and the response was strictly peptide-dependent. A comparison of the different Ltk⁻-HN clones revealed a correlation of the level of HN expression with the enhancement of specific CTL activity (Figs. 3 and 4). A quantitative comparison of the fluorescence intensity of the HN transfectants with that of cells infected with increasing amounts of NDV revealed that the 2 weakly staining clones Ltk⁻-HN.A3 and Ltk⁻HN.A4 were comparable to infection with 90 and 100 haemagglutinin units (HU)/10⁷ cells of NDV respectively, while that of clone, Ltk⁻-HN.A2 was equivalent to 200 (HU)/10⁷ cells. The range of amounts of HN expressed in the Ltk⁻-HN clones was thus within the optimal concentration seen with whole NDV infection in the tumour-specific immune response, P. von Hoegen et al., Invasion and Metastasis 9, 117-133 (1989). The functional analysis for CTL stimulatory capacity revealed that the expressed HN was slightly less effective than the intact virus used for infection, since Ltk⁻-HN.A2 cells were only comparable in lytic effect to the NDV infected Ltk⁻ cells [100 (HU/ml)], even though they showed higher HN expression and NA activity. The other 2 clones (Ltk⁻-HN.A3 and Ltk⁻-HN.A4), with only 2-fold increased relative fluorescence for HN-expression, showed a significant enhancement of CTL activity, indicating that relatively low amounts of expressed HN were sufficient to increase antigen-specific activation of CTL.

### Example 2

### General enhancement of CTL activity

To prove that the enhancing effect of NDV on the activation of CTL was not a specific phenomenon seen only with the influenza NP peptide, the effect of NDV infection of APCs in other antigen-specific CTL responses was examined in mixed leukocyte cell cultures. Different responses tested were (A) anti-ESb tumour antigen, (B) anti-minor H antigen, (C) anti allo-MHC antigen and (D) anti-influenza NP 50-63 peptide.

Although whole virus was used in these experiments, they include an experiment in which the HN cDNA-transfected Ltk⁻ cells were used, with similar result to when using whole virus. By reasonable inference, therefore, the Example demonstrates the likelihood that HN cDNA will be as effective as whole NDV in relation to those other antigen-specific APCs.

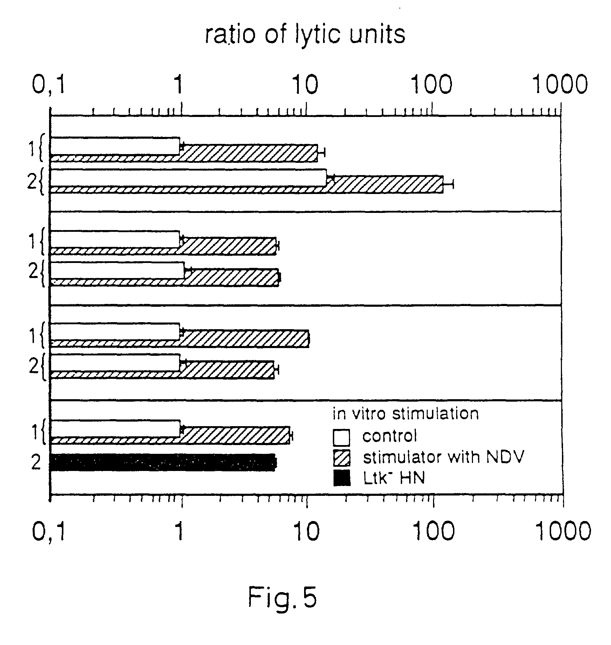

Mice were primed in vivo, and their spleen cells or tumour cells were removed after 9 days. The removed cells were subjected to a 5 day in vitro stimulation according to the Table below. The stimulated cells were used as they were (controls) or after infection with 100 HU/10⁷ cells of NDV, except in experiment D2 where the Ltk⁻ cells were transfected with the HN cDNA as described above. The cytotoxicity of these effector cells was determined in a 4-h ⁵¹Cr-release assay, whereby the specific target cells were ESb (A,B,C), EL-4 (C), and peptide-loaded Ltk⁻ cells (D). ESb cells are a spontaneous highly metastatic variant of the methylcholanthrene-induced lymphoma cell line Eb (Professor V. Schirrmacher, DKFZ, Heidelberg). EL-4 cells are available from the American Type Culture Collection (ATCC) as "TIB 39". The values are expressed as a "stimulation index" based on lytic units (LU)/10⁶ cells. One LU is the number of effector cells which cause 30% lysis of the target cells (5 x 10³). LU with stimulator cells without NDV modification were set at a stimulation index of 1. The results are shown in Fig. 5.

**Table:**

| protocol for stimulation of CTL | | | | |
|---|---|---|---|---|
| strains mice of | | priming (in vivo) | | stimulators * (in vitro) |
| A | DBA/2 | 1. | ESb | ESb |
| | | | | |
| | | 2. | ESb-NDV | ESb |
| B | B10.D2 | 1. | DBA/2 | DBA/2 (spleen cells) |
| | | | | |
| | | 2. | DBA/2-NDV | DBA/2 (spleen cells) |
| C | DBA/2 | 1. | - | C57BL/6 (spleen cells) |
| | | | | |
| | C57BL/6 | 2. | - | DBA/2 (spleen cells) |
| D | C3H | 1. | peptide NP 50-63 | Ltk⁻ + peptide NP 50-63 |
| | | 2. | | Ltk⁻ -HN.A2 + peptide NP 50-63 |

| | | | | |
|---|---|---|---|---|
| A anti-tumour (target cells:ESb) | | | | |
| B anti-minor H antigen (tc: ESb) | | | | |
| C anti-allo MHC antigen (tc: EL4, ESb) | | | | |
| D anti-peptide (tc: Ltk⁻+ peptide) | | | | |
| * each experiment A, B, C and D1 was carried out with and without infection of the stimulator cells by whole NDV. | | | | |

In the immune response of DBA/2 mice against the syngeneic ESb tumour (A) there was an 11-fold increase in CTL response, when NDV-infected ESb cells were used instead of ESb as stimulator cells in vitro. A similar augmentation could be seen upon in vivo immunisation (priming with NDV-infected ESb instead of ESb). When virus-infected tumour cells were used both for priming and restimulation, a synergism between the two enhancing effects was seen so that a more than 100-fold increase of the stimulation index was observed. In the minor-H response, no augmentation effect was observed upon priming with virus infected cells, which could be due to the fact that the immune response was already optimal. Optimal CTL responses in vitro could also not be augmented by NDV infection of stimulator cells (data not shown). The culture conditions were therefore limited to give stronger immune responses against minor-H (Fig. 5B) and alloantigens (Fig. 5C) by using only 10% of the standard stimulator cells. Under such sub-optimal conditions also in the minor-H and alloantigen responses a 6 fold increase in the CTL responses was observed upon virus infection of the stimulator cells.

## Claims

1. Use of a DNA coding for a HN protein of a paramyxovirus or a T-cell stimulation-enhancing peptide thereof, in each case free of native paramyxovirus and free of any associated specific antibody to the HN protein for the manufacture of a medicament for enhancing the antigen-specific stimulation of cytotoxic T-cell (CTL) responses in humans.

2. Use according to claim 1 wherein the paramyxovirus is Newcastle Disease Virus.

3. Use according to claim 1 wherein the HN protein has at least 70% amino acid identity with that of Newcastle Disease Virus strain Beaudette C.

4. Use according to claim 1 wherein the DNA coding for the HN protein has at least 70% identitidy with that of Newcastle Disease Virus strain Beaudette C.

5. Use according to any preceding claim wherein the antigen is a cancer antigen.

6. Use according to claim 5 wherein the cancer antigen is a tumor antigen.

7. A vaccine suitable for use in combatting a disease in a human patient, to which a T-cell mediated immue response can be mounted, the vaccine comprising antigen-presenting cells (APCs) tolerable to the patient, presenting an antigen of high specificity for the disease and containing a HN protein-providing component comprising a DNA coding for a HN protein of a paramyxovirus or a T-cell stimulation-enhancing peptide thereof, in each case free of native paramyxovirus and free of any associated specific antibody to the HN protein, whereby the in vivo stimulation of cyotoxic T-cells of high specificity for cytoloysis of diseased cells is increased by the HN protein-providing component.

8. A vaccine according to claim 7 wherein the APCs are provided by inactivated diseased cells previously removed from the patient to be treated or from a donor patient of matched MHC type.

9. A vaccine according to claim 7 wherein the APCs are macrophages, HLA class I expressing cells or HLA class II expressing cells.

## Patentansprüche

1. Verwendung einer DNA kodierend für ein HN-Protein eines Paramyxovirus oder ein T-Zell-Stimulation-verbesserndes Peptid davon, jedenfalls frei von nativem Paramyxovirus und frei von einem assoziierten spezifischen Antikörper für das HN-Protein, zur Herstellung eines Medikaments zur Verbesserung der antigen-spezifischen Stimulation von zytotoxischen T-Zell (CTL)-Antworten in Menschen.

2. Verwendung nach Anspruch 1, wobei der Paramyxovirus Newcastle Disease Virus ist.

3. Verwendung nach Anspruch 1, wobei das HN-Protein mindestens 70% Aminosäurenidentität mit dem von Newcastle Disease Virus Stamm Beaudette C hat.

4. Verwendung nach Anspruch 1, wobei die DNA kodierend für das HN-Protein mindestens 70% Identität mit der von Newcastle Disease Virus Stamm Beaudette C hat.

5. Verwendung nach einem der vorhergehenden Ansprüche, wobei das Antigen ein Krebsantigen ist.

6. Verwendung nach Anspruch 5, wobei das Krebsantigen ein Tumorantigen ist.

7. Vakzine geeignet zur Verwendung bei der Abwehr einer Erkrankung in einem menschlichen Patienten, in dem eine T-Zell vermittelte Immunantwort erzeugt werden kann, wobei die Vakzine für den Patienten verträgliche Antigenpräsentierende Zellen (APCs) enthält, ein Antigen hoher Spezifität für die Erkrankung präsentiert und eine HN-Protein bereitstellende Komponente enthält, welche eine DNA kodierend für ein HN-Protein eines Paramyxovirus oder ein T-Zell-Stimulation-verbesserndes Peptid davon, jedenfalls frei von nativem Paramyxovirus und frei von einem assoziierten spezifischen Antikörper für das HN-Protein, aufweist, wobei die in-vivo Stimulation zytotoxischer T-Zellen hoher Spezifität für die Zytolyse einer erkrankten Zelle durch die HN-Protein bereitstellende Komponente vergrößert ist.

8. Vakzine nach Anspruch 7, wobei die APCs durch inaktivierte erkrankte Zellen, die dem zu behandelnden Patienten vorher entnommen worden waren oder von einem Spenderpatienten mit passendem MHC-Typ bereitgestellt sind.

9. Vakzine nach Anspruch 7, wobei die APCs Makrophagen, HLA-Klasse I exprimierende Zellen oder HLA-Klasse II exprimierende Zellen sind.

## Revendications

1. Utilisation d'un ADN codant pour une protéine HN d'un paramyxovirus ou un de aes peptides amplificateurs de stimulation des lymphocytes T, dans chaque cas dépourvu de paramyxovirus naturel et dépourvu de tout anticorps spécifique associé contre la protéine HN, pour la production d'un médicament destiné à amplifier la stimulation, spécifique d'antigène, des réponses des lymphocytes T cytotoxiques (CTL) chez l'homme.

2. Utilisation suivant la revendication 1, dans laquelle le paramyxovirus est le virus de la maladie de Newcastle.

3. Utilisation suivant la revendication 1, dans laquelle la protéine HN présente une identité d'aminoacides d'au moins 70 % avec celle de la souche Beaudette C du virus de la maladie de Newcastle.

4. Utilisation suivant la revendication 1, dans laquelle l'ADN codant pour la protéine HN présente une identité d'au moins 70 % avec celui de la souche Beaudette C du virus de la maladie de Newcastle.

5. Utilisation suivant l'une quelconque des revendications précédentes, dans laquelle l'antigène est un antigène de cancer.

6. Utilisation suivant la revendication 5, dans laquelle l'antigène de cancer est un antigène tumoral.

7. Vaccin apte à l'utilisation pour combattre une maladie chez un patient humain, permettant d'engendrer une réponse immunitaire à médiation par les lymphocytes T, vaccin qui comprend des cellules présentatrices d'antigène (APC) aptes à la tolérance vis-à-vis du patient, présentant un antigène de grande spécificité pour la maladie et contenant un constituant de formation de protéine HN, comprenant un ADN codant pour une protéine HN d'un paramyxovirus ou un de ses peptides amplificateurs de stimulation des lymphocytes T, dans chaque cas dépourvu de paramyxovirus naturel et dépourvu de tout anticorps spécifique associé contre la protéine HN, la stimulation in vivo des lymphocytes T cytotoxiques de haute spécificité pour la cytolyse des cellules pathologiques étant ainsi accrue par le constituant de formation de protéine HN.

8. Vaccin suivant la revendication 7, dans lequel les APC sont fournies par des cellules pathologiques inactivées préalablement prélevés chez le patient à traiter ou chez un patient servant de donneur, ayant un type de complexe majeur d'histocompatibilité (CMH) concordant.

9. Vaccin suivant la revendication 7, dans lequel les APC sont des macrophages, des cellules d'expression de HLA de catégorie I ou des cellules d'expression de HLA de catégorie II.
